# DEMANDE DE BREVET EUROPEEN

(11) **EP 2 208 492 A1**
(43) Date de publication de la demande: **21.07.2010**
(21) Numéro de dépôt: 10354003.5
(22) Date de dépôt: 13.01.2010
(51) Int. Cl.: A61H 7/00, A61H 15/00, A61H 15/02, A61H 23/02, A61H 33/00, A61H 37/00

(54) **Baignoire ou cabine de douche équipée d'un dispositif de massage**

(30) Priorité: 16.01.2009 FR 0900196
(71) Demandeur: New Bath, 38340 Voreppe (FR)
(72) Inventeur: Aubourg, Désiré, 10150 Pont-Sainte-Marie (FR)
(74) Mandataire: Hecké, Gérard

(57) **Abrégé**

Un dispositif de massage comporte un bloc moteur d'entraînement accouplé à une série de boules 12 de massage installées à travers une ouverture 17 dans la paroi d'une baignoire 11 ou d'une cabine de douche. L'ouverture 17 de passage des boules 12 de massage est recouverte du côté intérieur par un film 20 d'étanchéité formé par un tissu transparent permettant la diffusion de la lumière ambiante ou la transmission d'un faisceau lumineux d'éclairage, et du côté extérieur par un revêtement 31 auxiliaire de glissement, lequel est constitué par un tissu lisse favorisant le glissement des boules de massage 12 rotatives. Tout contact direct des boules 12 avec le film 20 d'étanchéité interne est ainsi évité.

## Description

### Domaine technique de l'invention

L'invention est relative à un dispositif de massage pour une baignoire ou une cabine de douche, et comprenant
- un bloc moteur d'entraînement accouplé à une série de boules de massage installées à travers une ouverture dans la paroi de la baignoire ou de la cabine de douche,
- un film étanche recouvrant ladite ouverture du côté intérieur de la baignoire pour servir de joint d'étanchéité, ledit film étant destiné à se déformer lors de la mise en mouvement des boules pour simuler un massage par contact.

### État de la technique

Les documents US 5984883 et EP 1570827 se rapportent à des spas équipés de dispositifs de massage logés dans des ouvertures ménagées dans la paroi des spas. Les organes de massage mobiles agissent directement sur un film souple déformable. Le frottement des organes de massage sur le film souple nécessite de fréquentes opérations de maintenance suite à une usure prématurée du film d'étanchéité. Il est de plus difficile de faire varier la force de massage.

En balnéothérapie et en hydrothérapie; il est classique d'équiper une baignoire ou un spa, avec un système de pompe à eau et d'injection d'air sous pression pour provoquer une circulation forcée de remous dans la baignoire remplie d'eau. Il en résulte un massage vibratoire pour le corps de l'utilisateur. Le niveau de bruit rayonné est assez élevé, et la zone de contact des remous sur le corps de l'utilisateur reste toujours la même.

Il existe également des cabines de douche renfermant un mécanisme de brosses installé sur un coulisseau montant et descendant. L'utilisateur se fait ainsi masser le dos tout en prenant sa douche.

### Objet de l'invention

L'objet de l'invention consiste à réaliser un dispositif électromécanique pour simuler un massage Shiatsu dans une baignoire ou une cabine de douche avec une force de massage ajustable selon la morphologie de la personne.

Le dispositif de massage selon l'invention est **caractérisé en ce que** l'ouverture de passage des boules de massage est recouverte du côté intérieur par un film transparent permettant la diffusion de la lumière ambiante ou la transmission d'un faisceau lumineux d'éclairage, et du côté extérieur par un revêtement auxiliaire de glissement, lequel est constitué par un tissu lisse favorisant le glissement des boules de massage, et évitant tout contact direct des boules avec le film d'étanchéité interne.

On a ainsi la possibilité de se faire masser en prenant son bain. Il est également possible de se faire masser sans remplir sa baignoire, ou sans ouvrir l'arrivée d'eau de sa cabine de douche.

Selon un mode de réalisation préférentiel, le bloc moteur d'entraînement comporte un dispositif d'actionnement électromécanique logé dans un carter de protection fixé sur ladite ouverture du côté extérieur de la paroi, les boules de massage traversant l'ouverture en faisant légèrement saillie vers l'intérieur de la baignoire ou de la cabine, et en prenant appui sur le revêtement auxiliaire. Les boules de massage sont réparties par paire sur au moins une palette rotative entraînée par une liaison cinématique de transmission accouplée au bloc moteur.

Selon une caractéristique de l'invention, les boules de massage d'une même unité sont décalées l'une de l'autre par rapport à la palette rotative de manière à imprimer un mouvement ondulatoire sur le film pendant le mouvement de rotation des boules.

Le bloc moteur comporte de préférence un propulseur à basse tension, notamment un motoréducteur à vitesse variable.

Un système de chauffage infrarouge peut aussi être prévu avantageusement à l'intérieur des boules de massage pour chauffer les zones de contact à travers le film.

### Description sommaire des dessins

D'autres avantages et caractéristiques ressortiront plus clairement de la description qui va suivre d'un mode particulier de réalisation de l'invention donné à titre d'exemple non limitatif et représenté aux dessins annexés, dans lesquels :
- la figure 1 représente une vue en perspective de l'intérieur d'une baignoire équipée du dispositif de massage selon l'invention ;
- la figure 2 est une vue partielle de l'extérieur de la baignoire après montage du dispositif de massage ;
- la figure 3 montre une vue en perspective du bloc moteur avec deux paires de boules de massage ;
- la figure 4 est une vue de profil du bloc moteur installé sur la paroide la baignoire ;
- la figure 5 illustre une vue en perspective éclatée d'un premier mode de montage du dispositif de massage et des accessoires de fixation ;
- la figure 6 est une vue en perspective éclatée d'un deuxième mode d'assemblage du réalisation du dispositif de massage ;

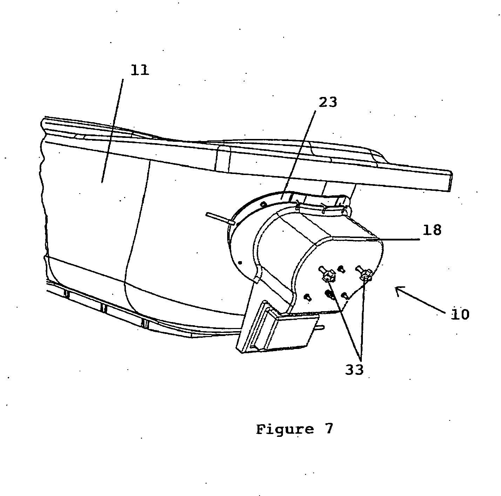

- la figure 7 représente l'ensemble baignoire et dispositif de massage après montage final du carter.

### Description d'un mode préférentiel de l'invention

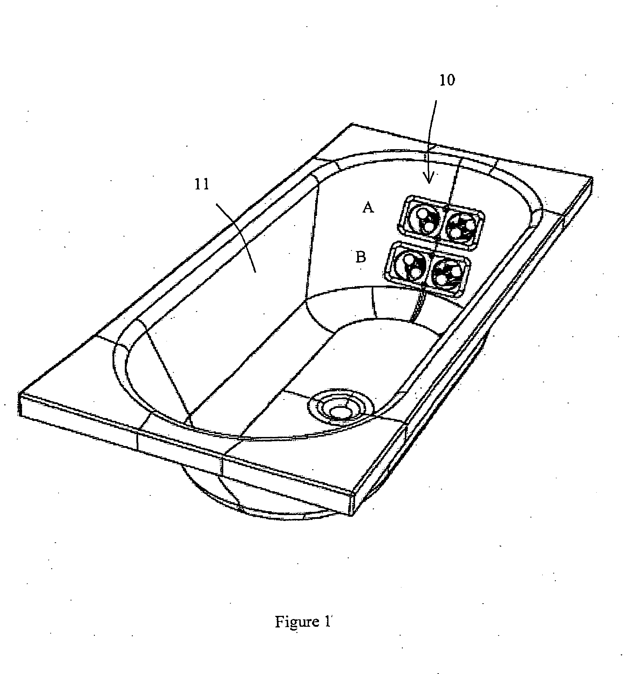

En référence aux figures 1 à 3, un dispositif d'actionnement 10 électromécanique est intégré dans une baignoire 11 pour constituer un bloc moteur réalisant un massage Shiatsu simulant un massage dorsale et lombaire avec contact sur la peau d'un utilisateur se trouvant à l'intérieur de la baignoire 11.

Le dispositif d'actionnement 10 électromécanique comporte une série de boules 12 sphériques de massage, entraînées en rotation à 360° par l'intermédiaire d'un propulseur 13 extérieur.

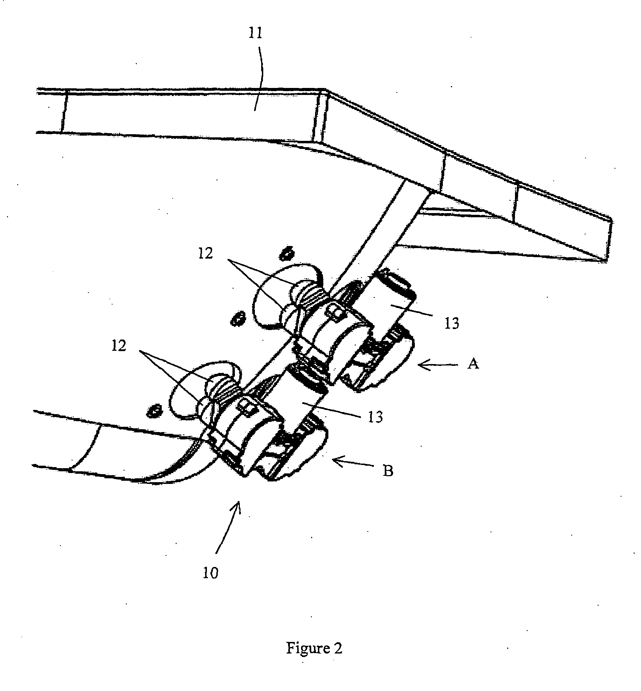

Les figures 1 et 2 montrent à titre d'exemple, une baignoire équipée d'un bloc moteur avec deux groupes A et B identiques de boules 12 de massage. Il est clair qu'un nombre différent de groupes de massage peut être utilisé en fonction du volume ou de la hauteur de la baignoire.

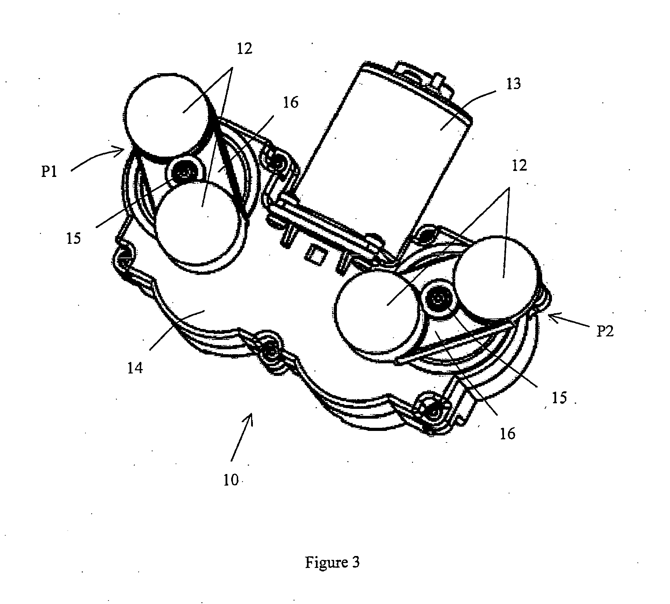

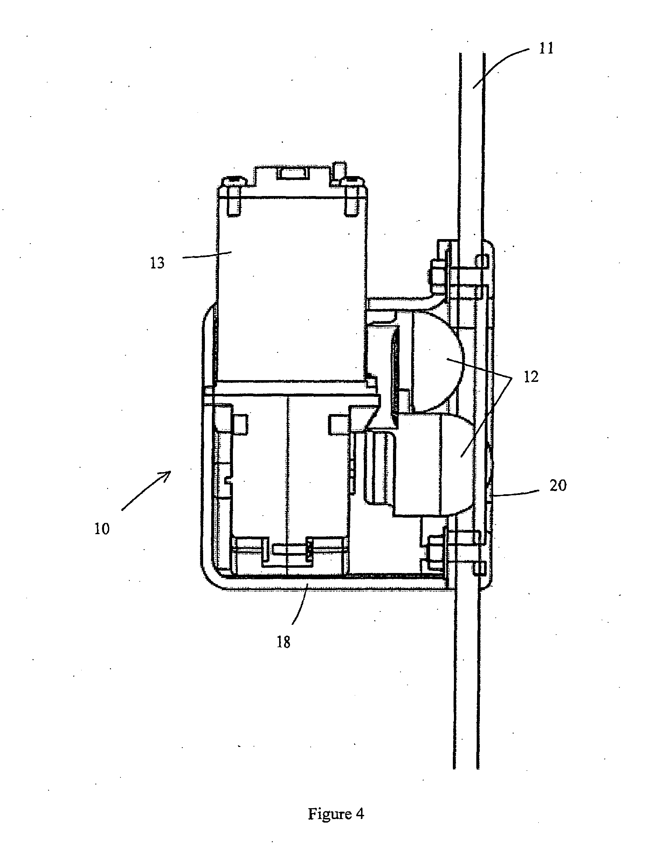

Par la suite, seul l'un des groupes sera décrit en détail en référence aux figures 3 et 4.

Le propulseur 13 de chaque groupe A ou B de massage peut être constitué par un motoréducteur à basse tension, ou tout autre moyen d'entraînement électromécanique à vitesse variable, accouplé par une liaison cinématique de transmission à deux paires P1, P2 de boules 12 de massage. L'ensemble est monté sur un support 14 monobloc fixé à la baignoire 11 du côté latéral extérieur.

Chaque paire P1, P2 de boules 12 de massage est portée par une palette 16 rotative, laquelle est montée à rotation autour d'un axe 15 intermédiaire associé à la liaison de transmission. Les deux axes 15 sont parallèles entre eux, et s'étendent perpendiculairement aux extrémités du support 14 de part et d'autre du propulseur 13 central.

On note sur la figure 4 que les deux boules 12 d'une même paire P1, P2 sont décalées l'une de l'autre dans la direction de l'axe 15 de manière à imprimer un mouvement ondulatoire sur le film 20 pendant la rotation de la palette 16.

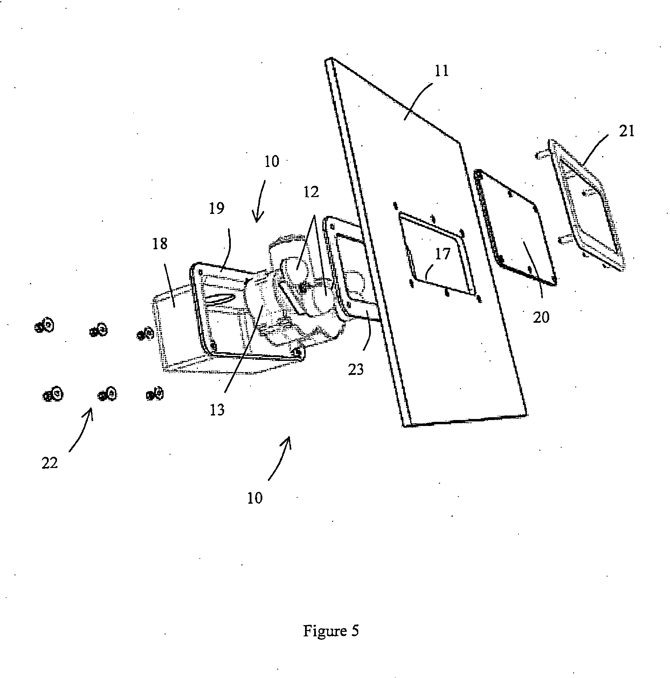

L'incorporation du dispositif d'actionnement 10 électromécanique dans la baignoire 11, est illustrée sur les figures 4 et 5. La paroi de la baignoire 11 est munie à cet effet d'une ouverture 17 débouchante, par exemple de forme rectangulaire, ou autre. L'intérieur de la baignoire ou paroi de la cabine est représenté à droite, et l'extérieur à gauche de la figure 5.

La présence de cette ouverture 17 de passage permet la mise en place du dispositif d'actionnement 10 électromécanique depuis l'extérieur de la baignoire 11. L'ensemble du bloc moteur avec les boules 12 de massage est logé dans un carter 18 de protection, lequel est pourvu d'un cadre 19 de fixation rectangulaire.

Les boules 12 de massage traversent l'ouverture 17 en faisant légèrement saillie vers l'intérieur de la baignoire 11, et en prenant appui sur un film 20 étanche et déformable. Ce dernier recouvre totalement l'ouverture 17 en étant plaqué contre la paroi interne de la baignoire 11 par un enjoliveur 21, et des vis de fixation 22.

Une platine 23 de fixation auxiliaire peut être intercalée entre le cadre 19 du carter 18 et la paroi extérieure de la baignoire 11.

Le film 20 est formé par un tissu technique à base d'élastomère, de caoutchouc ou de latex, présentant une faible épaisseur pour être facilement déformable lors du mouvement de rotation des boules 12 de massage. La déformation du film 20 simule réellement le contact des doigts sur la peau de l'utilisateur lorsque ce dernier est assis dans la baignoire 11.

L'utilisateur a la possibilité de se faire masser en prenant son bain, le film 20 en se déformant jouant en plus le rôle de joint dynamique garantissant l'étanchéité de la baignoire 11, et évitant toute projection d'eau.

En plus du massage Shiatsu exercé par la mise en mouvement des boules 12 sphériques, on peut rajouter un système de balnéothérapie standard par buses d'injection et pompe, ou par air pulsé.

Il est aussi possible de se faire masser sans devoir remplir la baignoire 11. Le film 20 peut être avantageusement transparent pour laisser passer la lumière ambiante ou un faisceau lumineux émis par une lampe extérieure, ou des diodes électroluminescentes.

Il est aussi possible d'intégrer un système de chauffage infrarouge à l'intérieur des boules 12 de massage pour chauffer les zones de massage à travers le film 20.

Le système de massage Shiatsu précédemment décrit peut aussi être appliqué à une cabine de douche (non représentée) en étant monté soit dans une ouverture de la paroi de la cabine, soit fixé sur un coulisseau mobile à mouvement vertical alternatif.

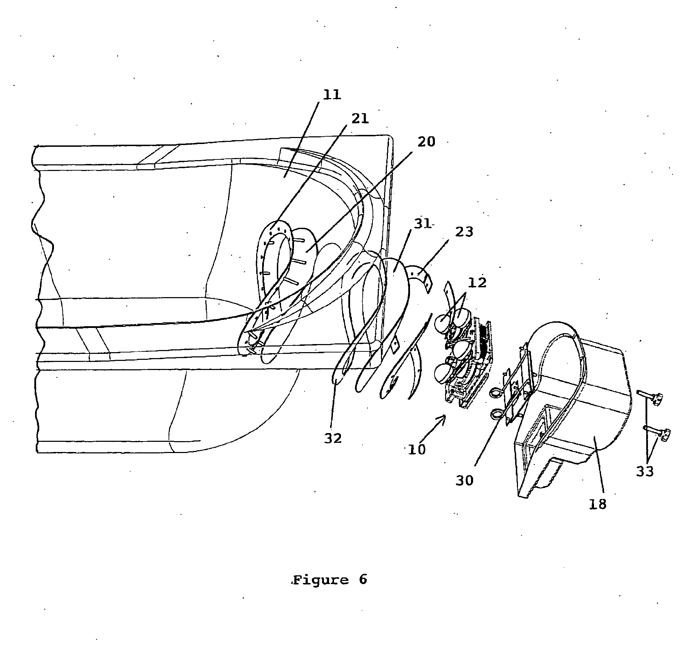

En référence à la figure 6, les mêmes numéros de repères seront utilisés pour désigner des pièces identiques ou similaires à celles du premier mode de réalisation de la figure 5. Le dispositif d'actionnement 10 motorisé à boules 12 rotatives de massage, est fixé sur une plaque support 30, l'ensemble étant logé dans le carter 18 à l'extérieur de la baignoire 11.

Le film 20 transparent d'étanchéité et son enjoliveur 21 obture l'ouverture 17 à l'intérieur de la baignoire 11, comme dans l'exemple de la figure 5. La présence du film 20 assure une étanchéité totale de la baignoire 11 quand elle est remplie d'eau, tout en laissant passer une lumière d'éclairage depuis l'extérieur vers l'intérieur.

L'ouverture de passage des boules de massage 12 dans la baignoire 11 est recouverte en plus du côté extérieur par un revêtement 31 auxiliaire de glissement, lequel est constitué par un tissu lisse favorisant le glissement des boules de massage 12 rotatives, et évitant le contact direct des boules 12 avec le film 20 d'étanchéité interne. Le revêtement 31 est ainsi placé entre les boules de massage 12 et le film 20 étanche.

Le carter 18 est solidarisé à la paroi extérieure de la baignoire 11 l'intermédiaire de la platine 23 de fixation auxiliaire, et d'un joint 32, le revêtement 31 de glissement étant intercalé entre le joint 32 et la platine 23. L'épaisseur du joint 32 permet de compenser les irrégularités de surface de la paroi de la baignoire. Il peut être réalisé en EPDM (éthylène propylène diène monomère), ou tout autre matière EPR (éthylène propylène rubber) présentant les mêmes caractéristiques que le caoutchouc naturel.

Des vis de réglage 33 sont accessibles sur le carter 18 pour assurer un déplacement relatif en translation de la plaque support 30 faisant varier la profondeur de pénétration des boules 12 à l'intérieur de la baignoire 11. La force de massage des boules 12 sur le corps peut ainsi être ajustée en fonction de la morphologie de la personne.

Selon une variante (non représentée), le dispositif d'actionnement électromécanique comporte une seule paire de boules de massage au lieu de quatre comme dans la figure 1.

Selon une autre variante, les boules de massage peuvent être actionnées par un système de piston mécanique accouplé à un moteur d'entraînement.

## Revendications

1. Baignoire ou cabine de douche équipée d'un dispositif de massage, lequel comporte :
- un bloc moteur d'entraînement accouplé à une série de boules (12) de massage installées à travers une ouverture (17) dans la paroi de la baignoire (11) ou de la cabine de douche,
- un film (20) étanche recouvrant ladite ouverture du côté intérieur de la baignoire pour servir de joint d'étanchéité, ledit film étant destiné à se déformer lors de la mise en mouvement des boules (12) pour simuler un massage par contact,
**caractérisée en ce que** l'ouverture (17) de passage des boules (12) de massage est recouverte du côté intérieur par ledit film (20) formé par un tissu transparent permettant la diffusion de la lumière ambiante ou la transmission d'un faisceau lumineux d'éclairage, et du côté extérieur par un revêtement (31) auxiliaire de glissement, lequel est constitué par un tissu lisse favorisant le glissement des boules de massage (12), et évitant tout contact direct des boules (12) avec le film (20) d'étanchéité interne.

2. Baignoire ou cabine de douche équipée d'un dispositif de massage selon la revendication 1, **caractérisée en ce que** le bloc moteur d'entraînement comporte un dispositif d'actionnement (10) électromécanique logé dans un carter (18) de protection accolé sur ladite ouverture du côté extérieur de la paroi, les boules (12) de massage traversant l'ouverture (17) en faisant légèrement saillie vers l'intérieur de la baignoire (11) ou de la cabine, et en prenant appui sur le revêtement (31) de glissement.

3. Baignoire ou cabine de douche équipée d'un dispositif de massage selon la revendication 2, **caractérisée en ce que** dispositif d'actionnement (10) motorisé à boules (12) rotatives de massage, est fixé sur une plaque support (30) ajustable pour faire varier la profondeur de pénétration des boules 12 à l'intérieur de la baignoire 11.

4. Baignoire ou cabine de douche équipée d'un dispositif de massage selon la revendication 3, **caractérisée en ce que** le carter (18) comporte des vis de réglage (33) de la force de massage par déplacement de ladite plaque support.

5. Baignoire ou cabine de douche équipée d'un dispositif de massage selon la revendications 2, **caractérisée en ce que** les boules (12) de massage sont réparties par paire (P1, P2) sur au moins une palette (16) rotative entraînée par une liaison cinématique de transmission accouplée au bloc moteur.

6. Baignoire ou cabine de douche équipée d'un dispositif de massage selon la revendications 5, **caractérisée en ce que** les boules (12) de massage d'une même paire (P1, P2) sont décalées l'une de l'autre par rapport à la palette (16) de manière à imprimer un mouvement ondulatoire sur le film (20) pendant le mouvement de rotation des boules (12).

7. Baignoire ou cabine de douche équipée d'un dispositif de massage selon l'une des revendications 1 à 6, **caractérisée en ce que** le bloc moteur comporte un propulseur à basse tension.

8. Baignoire ou cabine de douche équipée d'un dispositif de massage selon la revendications 7, **caractérisée en ce que** le propulseur est constitué par un motoréducteur.

9. Baignoire ou cabine de douche équipée d'un dispositif de massage selon l'une des revendications précédentes, **caractérisée en ce que** des moyens de chauffage infrarouge sont placés à l'intérieur des boules (12) de massage pour chauffer les zones de massage à travers le film (20).
